# EUROPEAN PATENT SPECIFICATION

(11) **EP 1 595 564 B1**
(45) Date of publication and mention of the grant of the patent: **02.11.2016**
(21) Application number: 05252826.2
(22) Date of filing: 09.05.2005
(51) Int. Cl.: A61M 11/06, A61M 16/12, A61M 16/14, A61M 16/06, A61M 15/00

(54) **Breathing device incorporating a nebulizer**
Beatmungsvorrichtung mit einem Vernebler
Dispositif respiratoire avec nébulisateur

(30) Priority: 10.05.2004 US 842334
(43) Date of publication of application: 16.11.2005
(73) Proprietor: Smiths Medical ASD, Inc., Plymouth, MN 55442 (US)
(72) Inventor: Niles, Rex A., Oneida, NY 13421 (US); Pelerossi, Richard K., Rome, NY 13440 (US); Richards, Fredrick M., Clinton, NY 13323 (US)
(74) Representative: Hackney, Nigel John

(56) References cited:
- EP-A1- 1 588 730
- WO-A-03/049791
- WO-A1-93/00952
- US-A- 3 667 463
- US-A- 3 874 379
- US-A- 4 767 576
- US-A- 6 041 776

## Description

This invention relates in general to an improved breathing system incorporating a nebulizer and, in particular, to an improved breathing system including a non-rebreather mask, a reservoir bag, a rescue valve and an improved nebulizer having an auxiliary gas port carried by a nebulizer head in a position removed from the nebulization chamber to introduce an auxiliary gas into the nebulizer head, when desired, without effecting the rate at which the breathing system applies medication to the nebulizer user.

Persons requiring treatment of certain kinds of respiratory conditions frequently need to have medications delivered directly to the lungs. Nebulized or aerosolized solutions are the preferred method of delivery of respiratory medication because the medicant is fragmented into small particles that are more efficiently deposited near sites of drug activity in the lungs.

US 3,874,379 discloses a manifold nebulizer system for use in a breathing circuit.

While nebulizers are well known to those skilled in the art, aerosolization of medications in a nebulizer is effected by putting a liquefied medication into a container or liquid reservoir and introducing a pressurized flow of gas through an aerosol nozzle carried within a nebulization chamber which is coupled to the liquefied medication contained in the liquid reservoir by a liquid draw tube. As the pressurized high velocity gas flows through the aerosol nozzle, the liquefied medication is drawn through the liquid draw or aspirator tube into the path of the high velocity gas and is fractured thereby into a mist, becoming entrained with the gas flow out of the nebulization chamber through the container and out therefrom through an output port.

In certain applications, nebulization therapy can be enhanced through the use of an auxiliary gas to supplement the nebulizing or driving gas being introduced through the aerosol nozzle. Such supplemental gases may be air, oxygen, helium or a combination of these two gases, referred to as heliox, depending upon the particular driving gas being used. The use of helium, an inert and metabolically stable gas which readily diffuses into swollen airways, or heliox, as a supplement to the nebulizing gas is also known, and these gasses are frequently used to drive the nebulized medication deeper into a user's lungs to deliver medications to bypass obstructed or restricted airways for a greater and more rapid effect from the medication.

US 3,667,463 discloses an aerosol generator provided with adaptor means to introduce additional oxygen to the aerosol to increase the oxygen concentration. US 4,767,576 discloses a nebulizer having a mixing chamber into which is projected a high velocity jet of oxygen to aspirate water. A compressed air input port the chamber projects a stream of compressed air adjacent to and alongside the oxygen jet.

WO93/00952 discloses a continuous flow nebulizing system for patients receiving long term nebulized medicant respiratory therapy.

One of the problems encountered when using supplemental gases, however, is that the injection of the supplemental gas into the nebulizing gas or into the nebulizing chamber affects the rate at which the liquefied medication passes out from the container through the aerosol output port. Generally, the rate at which the liquefied medication is to be nebulized and delivered to the user, is determined based upon, among other factors, the rate of flow and density of the driving or nebulizing gas being introduced into the nebulization chamber. When a supplemental gas is introduced into the nebulizing gas flow or into the nebulization chamber, the total quantity of medication delivered through the nebulization chamber changes. Accordingly, the predetermined rate at which the liquefied medication is discharged through the aerosol output port varies from that at which it was initially set.

Another problem encountered when a supplemental gas is introduced into a nebulizer breathing system, is that the supplemental gas is introduced at a point in the system close to the patient interface, such as a non-rebreathing (NRB) mask. The introduction of a supplemental gas at such a position relative to the NRB mask frequently results in the application of a non-uniform gas mixture. If a patient does not take uniform breaths, or the patient's breathing pauses, the percentage of the supplemental gas being introduced increases, or spikes, resulting in the patient inhaling a greater percentage of the supplemental gas with the next breath.

The present invention is directed to overcoming one or more of the problems or disadvantages associated with the relevant technology. As will be more readily understood and fully appreciated from the following detailed description of a preferred embodiment of the present invention, the invention is embodied in an improved nebulizer breathing system wherein a supplemental gas may be introduced into the nebulizer head at a position after the liquefied medication has been fractured or nebulized, so that the introduction of the supplemental gas does not effect the rate at which the nebulized liquefied medication is applied to the user, and the mixture of gases to be administered to a patient is added to the breathing system at a position removed from the point at which the gas mixture is administered to the patient.

The present invention provides a nebulizer breathing system according to claim 1 and a method according to claim 16.

Preferred embodiments are defined in the dependent claims.

Embodiments, aspects and examples disclosed herein, but not falling under the scope of claims 1 or 16, do not form part of the invention.

### Description of the Drawings

Further objectives of the invention, together with additional features contributing thereto and advantages accruing therefrom, will be apparent from the following description of a preferred embodiment of the invention which is shown in the accompanying drawings, wherein:
Fig. 1 illustrates an exploded view of the improved nebulizer breathing system of the invention; and
Fig. 2 is an elevational view of a component of the invention with portions broken away to better illustrate the internal construction thereof.

### Detailed Description of a Preferred Embodiment

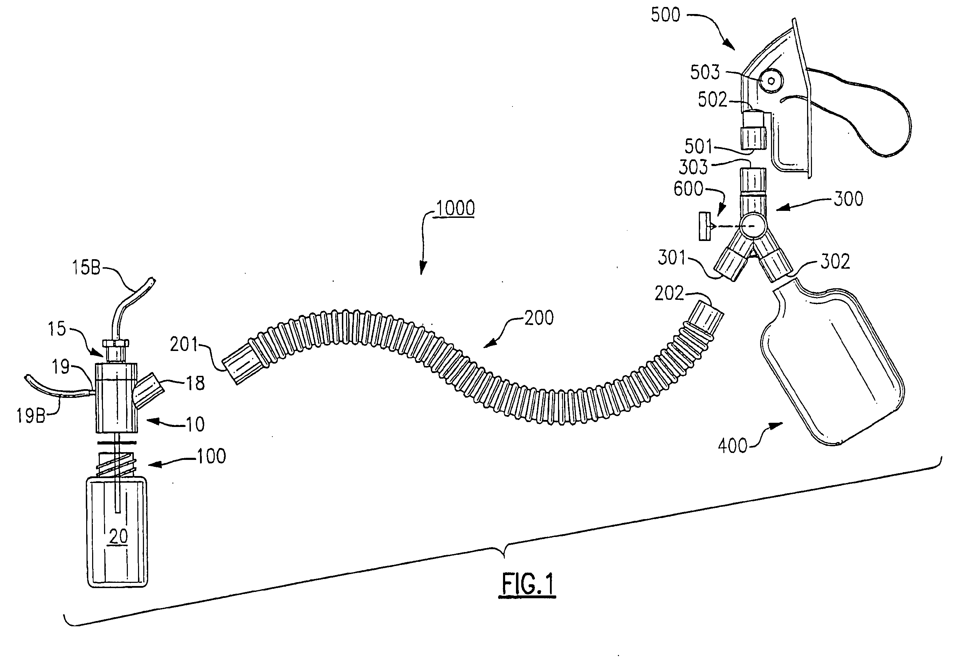

Referring now to the drawings, there is illustrated in Fig. 1 a preferred embodiment of the improved nebulizer breathing system 1000 which includes a nebulizer 100, a flexible tubing 200 connecting at a first inlet end to the output port of the nebulizer and at a second outlet end to the remainder of the breathing system, a wye 300 connected at a first input branch to the outlet end of the flexible tubing and having a second input branch and a discharge outlet, a flexible reservoir bag 400 having an outlet connected to the second input branch of the wye, a NRB mask 500 having an inlet connected to the discharge outlet of the wye, and a rescue valve 600 carried by the wye to permit a patient to inhale through the mask in the event the patient is beginning to draw a negative pressure or vacuum.

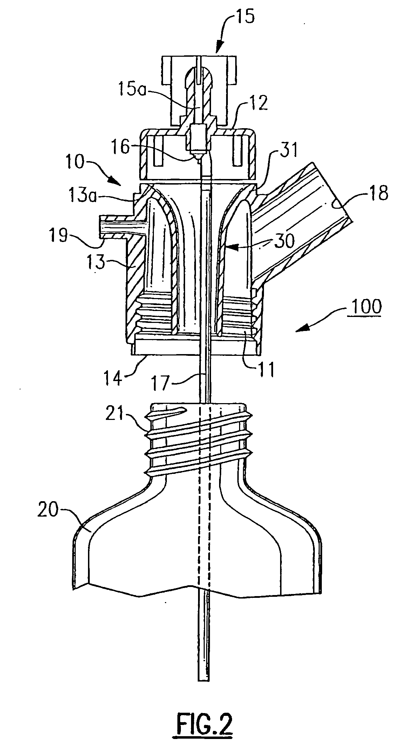

The nebulizer 100, illustrated in detail in Fig. 2, is preferably molded from translucent plastic and includes a nebulizer head 10 which is adapted to be received on a liquid container or receptacle 20. To this end, the lowermost portion of the nebulizer head 10 is open and formed with an internal thread 11 which is sized to receive a complementary external thread 21 formed on the top or neck portion of the liquid receptacle 20 for forming a liquid-tight seal.

The nebulizing head 10 has a closed circular-shaped top 12 and a depending skirt 13 forming a generally cylindrical shape with an open bottom 14 adapted to receive the liquid container 20. A nebulization chamber 30 is formed inside the nebulization head 10 and spaced downwardly from the top 12. The nebulization chamber 30 extends downwardly from a point of attachment 31 on the inner wall 13a of the skirt 13 towards the open end 14 of the nebulizer head, forming a chamber in which the liquid contained in the liquid container 20 is entrained in a driving or nebulizing gas introduced through the top 12 of the nebulizer head 10 into the nebulization chamber 30.

The top 12 of the nebulizer head 10 supports an adapter 15 by which a suitable source of driving or nebulizing gas is connected, such as by a flow meter or tubing 15b, for introduction into the nebulizing chamber 30. A conventional nebulizing nozzle 16 is supported within the nebulizing chamber 30 beneath the top 12, and is coupled to a passageway 15a formed through the adapter 15 by which the driving or nebulizing gas is introduced into the nebulizing chamber 30. The nebulizing nozzle 16 communicates with a suitable orifice at an upper terminal end of an aspirator tube 17 which extends downwardly into the liquid medicant contained in the liquid receptacle 20. In this manner, when a source of driving or nebulizing gas is coupled to the adapter 15, and introduced to the nebulizing nozzle 16, the liquid medicant contained within the receptacle 20 will be entrained in the driving or nebulizing gas and discharged out from the nebulizer chamber 30. The entrained aerosol so formed will then pass through the nebulizing head 10 to be discharged from the nebulizer 100 through a nebulized aerosol discharge outlet 18, formed through the sidewall 13, and into the flexible tubing 200.

To provide for the introduction of a supplemental gas into the nebulizer 100, an auxiliary gas inlet port 19 is formed in the side wall 13 at a point preferably opposite to the nebulized aerosol discharge outlet 18. The auxiliary gas inlet port 19 is coupled to a suitable source of supplemental gas, such as oxygen, helium or heliox, by tubing 19b and the inlet port extends into the nebulizing head 10, but not into the nebulizer chamber 30. In this manner the supplemental gas introduced through inlet port 19 will not affect the rate at which the aerosolized medicant is delivered from port 18. As heretofore described, when a supplemental gas is introduced into the driving or nebulizing gas, or the nebulizing chamber 30, the supplemental gas so introduced effects the uniformity of the administration of the medication. When a supplemental gas is so introduced, the rate of medication application increases with the increased gas flow. By introducing the supplemental gas through the auxiliary gas inlet port 19, the supplemental gas does not change the rate at which medication exiting the nebulizer through port 18 is applied to the nebulizer user. When a source of supplemental gas is not being introduced through auxiliary gas inlet port 19, a conventional cap, not shown, may be placed over the external opening thereto.

When the medication containing gas mixture exits the nebulizer 100 through the discharge or output port 18, the gas passes into the first inlet end 201 of the flexible tubing 200. The flexible tubing is preferably a collapsible type of flexible tubing such as that commercially available and sold under the trademark "POPPLE". The second outlet end 202 of the flexible tubing 200 is connected to a first input branch 301 of a wye connector 300 to space the output of the gas mixture from the nebulizer 100 a distance away from the inlet to a NRB mask 500 through which a patient breathes the medicant-containing mixture. Preferably, the flexible tubing 200 is at least approximately 18 inches (approximately 45·7 cm) in length, and can be approximately 72 inches (approximately 1·83 m) in length, to create a mixture that when inhaled by the user will be more uniform, than systems wherein a supplemental gas is administered to the user by means of a supplemental gas source connected to or substantially adjacent to the NRB mask 500, or connected to or substantially adjacent the wye connector 300.

A flexible bag reservoir 400 is connected in fluid communication to a second input branch 302 of the wye connector 300, and functions as an accumulator which holds enough of the incoming gas mixture to compensate for the patient's next breath in the event of over-breathing, as is known to those skilled in the respiratory care art.

The NRB mask 500 is sized to fit to the patient's face, and encompass the nose and mouth thereof. Such masks, or the equivalents thereof, are available in different sizes to accommodate patients from pediatric through adults. The mask 500 has an inlet 501 connected to a discharge outlet 303 of the wye 300. Conventional one-way inhalation 502 and one-way exhalation valves 503, one of which is shown, are carried in the NRB mask. The inhalation valve 502 is open during patient inhalation, and closes during patient exhalation. However, during periods of patient inactivity, the inhalation valve 501 may stay open because of the positive pressure coupled thereto by the nebulizer 100. The valve, however, prevents exhaled breath from traveling back into the wye 300. The exhalation valves 503 open during exhalation, and close during inhalation. In this manner, the expiratory air discharged by a patient is exhausted to atmosphere, and does not go back into the breathing system.

In order to ensure that a patient does not draw a negative pressure in the system in the event the patient is over-breathing, a conventional rescue valve 600 is incorporated into the wye 300. The rescue valve 600 is a one-way inhalation valve which is normally closed, but when a patient over-breathes the nebulizer system, the rescue valve 600 will open permitting the patient to breathe ambient air through the rescue valve, and preventing the patient from drawing a negative pressure or vacuum. When the patient is normally breathing, and does not over breath the system, the rescue valve 600 will remain closed.

While this invention has been described in the specification and illustrated in the drawings with reference to a preferred embodiment, it is intended that the invention not be limited to the particular embodiment disclosed in the specification and shown in the drawings as the best mode presently known by the inventors for carrying out this invention, nor confined to the details set forth, but that the invention will include all embodiments, modifications and changes that fall within the scope of the following claims:

## Claims

1. A nebulizer breathing system comprising:
a nebulizer (100) having a nebulizer head (10) adapted to be received on a receptacle adapted to contain a liquid medicant to be nebulized;
said nebulizer head (10) including a first chamber having a closed top (12), enclosing sides (13) and an open bottom (14) adapted to receive for coupling thereto a receptacle adapted to contain a liquid medicant to be dispensed at a predetermined rate of concentration;
said nebulizer head (10) including a second nebulizing chamber (30) carried within said first chamber at a position spaced from said closed top and extending downwardly therefrom;
said nebulizing chamber (30) having an open bottom adapted to pass entrained and nebulized liquid medicant outwardly therethrough;
a nebulizing nozzle (16) in fluid communication with said nebulizing chamber for creating a nebulized aerosol from liquid medicant coupled thereto;
means for coupling a flow of nebulizing gas into said first chamber to said nebulizing nozzle and through said second nebulizing chamber carried within said first chamber;
an aspirator tube (17) having a first end positioned in fluid communication adjacent to said nebulizing nozzle (16), and a second end positioned to be received into liquid medicant contained within the receptacle;
an auxiliary gas inlet port (19) configured to introduce a supplemental gas into said first chamber extending into said first chamber at a position removed from the interior of said nebulizing chamber (30);
said first chamber having a discharge outlet (18) spaced from said open bottom of said nebulizing chamber (30) and said auxiliary gas inlet port (19), for discharging liquid medicant entrained in said nebulizing chamber and the supplemental gas introduced into said first chamber through said auxiliary gas inlet port (19) from said nebulizer; and
a flexible tube (200) coupled to said nebulizer discharge outlet (18) and extending there from, said flexible tube having a discharge outlet (202) for connecting the entrained liquid medicant discharged from said nebulizer discharge outlet to a patient interface;
said flexible tube (200) extending a length of at least approximately 45.7cm (18 inches) to provide a substantially uniform entrained liquid medicant discharge to the patient interface.

2. The nebulizer breathing system of claim 1 further comprising said patient interface, said patient interface comprises a non-rebreathing mask (500) for receiving the entrained liquid medicant discharged from said flexible tube (200).

3. The nebulizer breathing system of Claim 2 wherein said non-rebreathing mask (500) includes a valve (502) which opens upon inhalation and closes upon exhalation, and a valve which opens upon exhalation and closes upon inhalation (503).

4. The nebulizer breathing system of Claim 2 further including a reservoir (400) for accumulating a quantity of entrained liquid medicant discharged from said flexible tube for discharge to said non-rebreathing mask in response to a patient's breathing.

5. The nebulizer breathing system of Claim 2 further including a normally closed rescue valve (600) which is opened in response to a patient over-breathing the quantity of the entrained liquid medicant discharged from said flexible tube to said non-rebreathing mask.

6. The nebulizer breathing system of Claim 2 further including:
a wye (300) having a first inlet (301) connected to the outlet of said flexible tube (200), a second inlet (302) and a discharge outlet (303);
a reservoir (400) for accumulating a quantity of entrained liquid medicant discharged from said flexible tube, said reservoir being connected to said second inlet of said wye; and
said non-rebreathing mask (500) being connected to said discharge outlet (303) of said wye.

7. The nebulizer breathing system of Claim 6 wherein said wye further includes a normally closed rescue valve (600) which is opened in response to a patient over-breathing the quantity of entrained liquid medicant discharged from said flexible tube to said no-rebreathing mask.

8. The nebulizer breathing system of Claim 1 wherein said auxiliary gas inlet port and said discharge outlet are in opposed positions relative to each other, and said nebulizing chamber carried within said first chamber extends therebetween.

9. The nebulizer breathing system of claim 1 further including said receptacle (20) for containing liquid medicant to be dispensed through said discharge outlet.

10. The nebulizer breathing system of Claim 9 wherein said receptacle includes means for releasibly connecting said receptacle to said first chamber in sealing engagement therewith.

11. The nebulizer breathing system of Claim 8 wherein said nebulizing chamber has an open top attached to the interior of said first chamber at a position above said auxiliary gas inlet port and said nebulizing chamber (30) extends downwardly therefrom such that said open bottom of said nebulizing chamber is positioned at a location removed from said auxiliary gas inlet port.

12. The nebulizer breathing system of Claim 11 wherein said nebulizing nozzle (16) is supported from said first chamber top above said nebulizing chamber for entraining liquid medicant communicated thereto through said aspirator tube.

13. The nebulizer breathing system of claim 8 wherein said nebulizing chamber extends downwardly within said first chamber such that said open bottom of said nebulizing chamber (30) is positioned at a location below said auxiliary gas inlet port and below said first chamber discharge outlet.

14. The nebulizer breathing system of Claim 1 wherein said means for coupling a flow of nebulizing gas into said first chamber comprises an adapter for releasably coupling a source of driving gas to said nebulizing nozzle (16).

15. The nebulizer breathing system of claim 1 wherein said auxiliary gas inlet port includes means for selectively closing said auxiliary gas inlet port when not in use.

16. A method of delivering a liquid medicant entrained in a selected mixture of gases in a substantially uniform manner, comprising:
passing a flow of driving gas into a nebulizer chamber of a nebulizer for fracturing medicant contained therein and creating a medicant aerosol in said nebulizer chamber;
introducing a supplemental gas into said nebulizer at a location removed from said nebulizer chamber thereby mixing said supplemental gas with said medicant aerosol from said nebulizer chamber, without affecting the rate at which medicant is delivered from said nebulizing chamber to a breathing mask;
discharging said mixture of said medicant aerosol nebulized by said driving gas in said nebulizer chamber, and said supplemental gas, from said nebulizer into a flexible tube; and
passing said aerosolized medicant mixture through said flexible tube to said breathing mask for a distance of at least approximately 45.7cm (18 inches) to maintain a substantially uniform mixture of aerosolized medicant at said breathing mask.

## Patentansprüche

1. Verneblerbeatmungssystem, umfassend:
einen Vernebler (100) mit einem Verneblerkopf (10), der geeignet ist, auf einem Behälter aufgenommen zu werden, der geeignet ist, ein zu zerstäubendes, flüssiges Arzneimittel zu enthalten;
wobei der Verneblerkopf (10) eine erste Kammer umfasst, die eine geschlossene Oberseite (12) umschließende Seiten (13) und einen offenen Boden (14) aufweist, der geeignet ist, zum Koppeln mit dieser einen Behälter aufzunehmen, der geeignet ist, ein flüssiges Arzneimittel zu enthalten, das bei einer vorbestimmten Konzentrationsrate abgeben wird;
wobei der Verneblerkopf (10) eine zweite Vernebelungskammer (30) aufweist, die in der ersten Kammer in einer von der geschlossenen Oberseite beabstandet angeordneten Position und sich davon nach unten erstreckend aufgenommen ist;
wobei die Vernebelungskammer (30) einen offenen Boden aufweist, der geeignet ist, ein mitgerissenes und zerstäubtes, flüssiges Arzneimittel durch diese hindurch nach außen zu leiten;
eine Vernebelungsdüse (16) in Fluid-Kommunikation mit der Vernebelungskammer zum Erzeugen eines zerstäubten Aerosols aus damit gekoppeltem, flüssigem Arzneimittel;
Mittel zum Koppeln eines Stromes aus Vernebelungsgas in die erste Kammer zu der Vernebelungsdüse und durch die zweite Vernebelungskammer hindurch, die innerhalb der ersten Kammer aufgenommen ist;
ein Aspiratorrohr (17) mit einem ersten Ende, das in Fluid-Kommunikation angrenzend an die Vernebelungsdüse (16) positioniert ist, und einem zweiten Ende, das positioniert ist, in flüssigem Arzneimittel aufgenommen zu werden, das in dem Behälter enthalten ist;
eine Hilfsgaseinlassöffnung (19), die konfiguriert ist, ein Zusatzgas in die erste Kammer einzubringen, die sich in die erste Kammer an einer vom Inneren der Vernebelungskammer (30) entfernten Stelle erstreckt;
wobei die erste Kammer einen Ausstoßauslass (18), der von dem offenen Boden der Vernebelungskammer (30) beabstandet angeordnet ist, und die Hilfsgaseinlassöffnung (19) aufweist, um das in der Vernebelungskammer mitgerissene flüssige Arzneimittel und das in die erste Kammer durch die Hilfsgaseinlassöffnung (19) eingebrachte Zusatzgas aus dem Vernebler auszustoßen; und
einen elastischen Schlauch (200), der mit dem Verneblerausstoßauslass (18) gekoppelt ist und sich von diesem erstreckt, wobei der elastische Schlauch einen Ausstoßauslass (202) zum Verbinden des aus dem Verneblerausstoßauslass ausgestoßenen, mitgerissenen flüssigen Arzneimittels mit einer Patientenschnittstelle aufweist;
wobei sich der elastische Schlauch (200) über eine Länge von mindestens ungefähr 45,7 cm (18 Zoll) erstreckt, um der Patientenschnittstelle ein im Wesentlichen gleichmäßiges Ausstoßen eines mitgerissenen flüssigen Arzneimittels bereitzustellen.

2. Verneblerbeatmungssystem nach Anspruch 1, ferner umfassend die Patientenschnittstelle,
wobei die Patientenschnittstelle eine Nicht-Rückatmungsmaske (500) zur Aufnahme des mitgerissenen flüssigen Arzneimittels umfasst, das aus dem elastischen Schlauch (200) ausgestoßen wird.

3. Verneblerbeatmungssystem nach Anspruch 2, worin die Nicht-Rückatmungsmaske (500) ein Ventil (502) umfasst, das sich beim Einatmen öffnet und beim Ausatmen schließt, und ein Ventil, das sich beim Ausatmen öffnet und beim Einatmen schließt (503).

4. Verneblerbeatmungssystem nach Anspruch 2, ferner umfassend ein Reservoir (400) für das Ansammeln einer mitgerissenen flüssigen Arzneimittelmenge, die aus dem elastischen Schlauch ausgestoßen wird, um in die Nicht-Rückatmungsmaske als Antwort auf das Atmen eines Patienten ausgestoßen zu werden.

5. Verneblerbeatmungssystem nach Anspruch 2, ferner umfassend ein normal geschlossenes Rettungsventil (600), das als Antwort auf eine Patienten-Überatmung mit der mitgerissenen flüssigen Arzneimittelmenge, die aus dem elastischen Schlauch in die Nicht-Rückatmungsmaske ausgestoßen wird, geöffnet wird.

6. Verneblerbeatmungssystem nach Anspruch 2, ferner umfassend:
ein Y-Rohr (300), das einen ersten Einlass (301), der mit dem Auslass des elastischen Schlauchs (200) verbunden ist, und einen zweiten Einlass (302) und einen Ausstoßauslass (303) aufweist;
ein Reservoir (400) zum Ansammeln einer mitgerissenen flüssigen Arzneimittelmenge, die aus dem elastischen Schlauch ausgestoßen wird, wobei das Reservoir mit dem zweiten Einlass des Y-Rohrs verbunden ist; und
wobei die Nicht-Rückatmungsmaske (500) mit dem Ausstoßauslass (303) des Y-Rohrs verbunden ist.

7. Verneblerbeatmungssystem nach Anspruch 6, worin das Y-Rohr ferner ein normal geschlossenes Rettungsventil (600) umfasst, das als Antwort auf eine Patienten-Überatmung mit der mitgerissenen flüssigen Arzneimittelmenge, die aus dem elastischen Schlauch in die Nicht-Rückatmungsmaske ausgestoßen wird, geöffnet wird.

8. Verneblerbeatmungssystem nach Anspruch 1, worin die Hilfsgaseinlassöffnung und der Ausstoßauslass im Verhältnis zueinander in entgegengesetzten Positionen angeordnet sind, und die in der ersten Kammer aufgenommene Vernebelungskammer sich dazwischen erstreckt.

9. Verneblerbeatmungssystem nach Anspruch 1, ferner umfassend den Behälter (20) zum Enthalten des flüssigen Arzneimittels, das durch den Ausstoßauslass abzugeben ist.

10. Verneblerbeatmungssystem nach Anspruch 9, worin der Behälter ein Mittel für das lösbare Verbinden des Behälters mit der ersten Kammer umfasst, die in abdichtendem Eingriff mit dieser ist.

11. Verneblerbeatmungssystem nach Anspruch 8, worin die Vernebelungskammer eine offene Oberseite aufweist, die am Inneren der ersten Kammer an einer Stelle oberhalb der Hilfsgaseinlassöffnung befestigt ist, und die Vernebelungskammer (30) sich von dieser nach unten derart erstreckt, dass der offene Boden der Vernebelungskammer an einer Stelle positioniert ist, die von der Hilfsgaseinlassöffnung entfernt angeordnet ist.

12. Verneblerbeatmungssystem nach Anspruch 11, worin die Verneblerdüse (16) von der Oberseite der ersten Kammer oberhalb der Vernebelungskammer gelagert ist, um das zu dieser übermittelte flüssige Arzneimittel durch das Aspiratorrohr mitzureißen.

13. Verneblerbeatmungssystem nach Anspruch 8, worin sich die Vernebelungskammer innerhalb der ersten Kammer derart nach unten erstreckt, dass der offene Boden der Vernebelungskammer (30) an einer Stelle unterhalb der Hilfsgaseinlassöffnung und unterhalb des Ausstoßauslasses der ersten Kammer positioniert ist.

14. Verneblerbeatmungssystem nach Anspruch 1, worin das Mittel zum Koppeln eines Verneblergasstromes in die erste Kammer einen Adapter für ein lösbares Koppeln einer Treibgasquelle mit der Verneblerdüse (16) umfasst.

15. Verneblerbeatmungssystem nach Anspruch 1, worin die Hilfsgaseinlassöffnung ein Mittel für das wahlweise Schließen der Hilfsgaseinlassöffnung bei Nicht-Benutzung umfasst.

16. Verfahren für das Zuführen eines flüssigen Arzneimittels, das in einer ausgewählten Mischung von Gasen in einer im Wesentlichen gleichmäßigen Art und Weise mitgerissen wird, umfassend:
Einleiten eines Treibgasstroms in eine Vernebelungskammer eines Verneblers, um das darin enthaltene Arzneimittel aufzubrechen und ein Arzneimittel-Aerosol in der Vernebelungskammer zu erzeugen;
Einbringen eines Zusatzgases in den Vernebler an einer Stelle, die von der Vernebelungskammer entfernt angeordnet ist, wodurch das Zusatzgas mit dem Arzneimittel-Aerosol aus der Vernebelungskammer vermischt wird, ohne sich auf die Geschwindigkeit, bei der das Arzneimittel aus der Vernebelungskammer einer Beatmungsmaske zugeführt wird, auszuwirken;
Ausstoßen der Mischung des Arzneimittel-Aerosols, das durch das Treibgas in der Vernebelungskammer zerstäubt wird, und des Zusatzgases aus dem Vernebler in einen elastischen Schlauch hinein;
und
Einleiten der aerosolisierten Arzneimittelmischung durch den elastischen Schlauch hindurch in die Beatmungsmaske über eine Entfernung von mindestens ungefähr 45,7 cm (18 Zoll), um eine im Wesentlichen gleichmäßige Mischung eines aerosolisierten Arzneimittels bei der Beatmungsmaske aufrechtzuerhalten.

## Revendications

1. Système respiratoire avec nébulisateur comprenant :
un nébulisateur (100) ayant ayant une tête de nébulisateur (10) adaptée pour être reçue sur un récipient adapté pour contenir un médicament liquide à nébuliser ;
ladite tête de nébulisateur (10) ayant une première chambre ayant une partie supérieure fermée (12), enfermant des côtés (13) et un fond ouvert (14) adapté pour recevoir, pour son couplage, un récipient adapté pour contenir un médicament liquide à distribuer à un taux de concentration prédéterminé ;
ladite tête de nébulisateur (10) comprenant une seconde chambre de nébulisation (30) portée à l'intérieur de ladite première chambre à une position espacée de ladite partie supérieure fermée et s'étendant vers le bas à partir de cette dernière ;
ladite chambre de nébulisation (30) ayant un fond ouvert adapté pour laisser passer le médicament liquide entraîné et nébulisé à travers ce dernier vers l'extérieur ;
une buse de nébulisation (16) en communication de fluide avec ladite chambre de nébulisation pour créer un aérosol nébulisé à partir du médicament liquide, couplée à cette dernière ;
des moyens pour coupler un écoulement de gaz de nébulisation dans ladite première chambre, à ladite buse de nébulisation et à travers ladite seconde chambre de nébulisation portée à l'intérieur de ladite première chambre ;
un tube d'aspirateur (17) ayant une première extrémité positionnée en communication de fluide, adjacente à ladite buse de nébulisation (16) et une seconde extrémité positionnée pour être reçue dans le médicament liquide contenu à l'intérieur du récipient ;
un orifice d'entrée de gaz auxiliaire (19) configuré pour introduire un gaz supplémentaire dans ladite première chambre s'étendant dans ladite première chambre dans une position retirée de l'intérieur de ladite chambre de nébulisation (30) ;
ladite première chambre ayant une sortie de décharge (18) espacée dudit fond ouvert de ladite chambre de nébulisation (30) et dudit orifice d'entrée de gaz auxiliaire (19), pour décharger le médicament liquide entraîné dans ladite chambre de nébulisation et le gaz supplémentaire introduit dans ladite première chambre par ledit orifice d'entrée de gaz auxiliaire (19) à partir dudit nébulisateur ; et
un tube flexible (200) couplé à ladite sortie de décharge de nébulisateur (18) et s'étendant à partir de ce dernier, ledit tube flexible ayant une sortie de décharge (202) pour raccorder le médicament liquide entraîné, déchargé par ladite sortie de décharge de nébulisation, à une interface de patient ;
ledit tube flexible (200) ayant une longueur d'approximativement 45,7 cm (18 pouces) pour fournir une décharge de médicament liquide entraîné sensiblement uniforme à l'interface de patient.

2. Système respiratoire avec nébulisateur selon la revendication 1, comprenant en outre ladite interface de patient, ladite interface de patient comprend un masque sans réinspiration (500) pour recevoir le médicament liquide entraîné déchargé par ledit tube flexible (200).

3. Système respiratoire avec nébulisateur selon la revendication 2, dans lequel ledit masque sans réinspiration (500) comprend une valve (502) qui s'ouvre suite à l'inspiration et se ferme suite à l'expiration, et une valve qui s'ouvre suite à l'expiration et se ferme suite à l'inspiration (503).

4. Système respiratoire avec nébulisateur selon la revendication 2, comprenant en outre un réservoir (400) pour accumuler une quantité de médicament liquide entraîné déchargé par ledit tube flexible pour se décharger dans ledit masque sans réinspiration en réponse à une respiration du patient.

5. Système respiratoire avec nébulisateur selon la revendication 2, comprenant en outre une valve de secours normalement fermée (600) qui s'ouvre en réponse à un patient qui inspire de façon excessive la quantité de médicament liquide entraîné déchargé dudit tube flexible audit masque sans réinspiration.

6. Système respiratoire avec nébulisateur selon la revendication 2, comprenant en outre :
un raccord en étoile (300) ayant une première entrée (301) raccordée à la sortie dudit tube flexible (200), une seconde entrée (302) et une sortie de décharge (303) ;
un réservoir (400) pour accumuler une quantité de médicament liquide entraîné déchargé par ledit tube flexible, ledit réservoir étant raccordé à ladite seconde entrée dudit raccord en étoile ; et
ledit masque sans réinspiration (500) étant raccordé à ladite sortie de décharge (303) dudit raccord en étoile.

7. Système respiratoire avec nébulisateur selon la revendication 6, dans lequel ledit raccord en étoile comprend en outre une valve de secours normalement fermée (600) qui s'ouvre en réponse à un patient qui inspire de manière excessive la quantité de médicament liquide entraîné déchargé dudit tube flexible audit masque sans réinspiration.

8. Système respiratoire avec nébulisateur selon la revendication 1, dans lequel ledit orifice d'entrée de gaz auxiliaire et ladite sortie de décharge sont dans des positions opposées l'un par rapport à l'autre, et ladite chambre de nébulisation portée à l'intérieur de la première chambre s'étend entre eux.

9. Système respiratoire avec nébulisateur selon la revendication 1, comprenant en outre ledit récipient (20) pour contenir le médicament liquide à distribuer par ladite sortie de décharge.

10. Système respiratoire avec nébulisateur selon la revendication 9, dans lequel ledit récipient comprend des moyens pour raccorder de manière amovible ledit récipient à ladite première chambre en mise en prise étanche avec cette dernière.

11. Système respiratoire avec nébulisateur selon la revendication 8, dans lequel ladite chambre de nébulisation a une partie supérieure ouverte fixée à l'intérieur de ladite première chambre dans une position au-dessus dudit orifice d'entrée de gaz auxiliaire et ladite chambre de nébulisation (30) s'étend vers le bas à partir de cette dernière, de sorte que ledit fond ouvert de ladite chambre de nébulisation est positionné dans un emplacement retiré dudit orifice d'entrée de gaz auxiliaire.

12. Système respiratoire avec nébulisateur selon la revendication 11, dans lequel ladite buse de nébulisation (16) est supportée par ladite première chambre au-dessus de ladite chambre de nébulisation pour entraîner le médicament liquide en communication par le biais de cette dernière avec ledit tube d'aspirateur.

13. Système respiratoire avec nébulisateur selon la revendication 8, dans lequel ladite chambre de nébulisation s'étend vers le bas à l'intérieur de ladite première chambre de sorte que ledit fond ouvert de ladite chambre de nébulisation (30) est positionné à un emplacement au-dessous dudit orifice d'entrée de gaz auxiliaire et au-dessous de ladite sortie de décharge de la première chambre.

14. Système respiratoire avec nébulisateur selon la revendication 1, dans lequel lesdits moyens pour coupler un écoulement de gaz de nébulisation dans ladite première chambre comprennent un adaptateur pour coupler de manière amovible une source de gaz d'entraînement à ladite buse de nébulisation (16).

15. Système respiratoire avec nébulisateur selon la revendication 1, dans lequel ledit orifice d'entrée de gaz auxiliaire comprend des moyens pour fermer sélectivement ledit orifice d'entrée de gaz auxiliaire lorsqu'il n'est pas utilisé.

16. Procédé pour distribuer un médicament liquide entraîné dans un mélange sélectionné de gaz d'une manière sensiblement uniforme comprenant les étapes consistant à :
faire passer un écoulement de gaz d'entraînement dans une chambre nébulisation d'un nébulisateur pour fracturer le médicament contenu à l'intérieur de ce dernier et créer un aérosol de médicament dans ladite chambre de nébulisation ;
introduire un gaz supplémentaire dans ledit nébulisateur à un emplacement retiré de ladite chambre de nébulisation, mélangeant ainsi ledit gaz supplémentaire avec ledit aérosol de médicament à partir de ladite chambre de nébulisation, sans affecter la vitesse à laquelle le médicament est distribué de ladite chambre de nébulisation à un masque respiratoire ;
décharger ledit mélange dudit aérosol de médicament nébulisé par ledit gaz d'entraînement dans ladite chambre de nébulisateur, et ledit gaz supplémentaire, dudit nébulisateur dans un tube flexible ; et
faire passer ledit mélange de médicament en aérosol par ledit tube flexible jusqu'audit masque respiratoire sur une distance d'au moins approximativement 45,7 cm (18 pouces) pour maintenir un mélange sensiblement uniforme de médicament sous forme d'aérosol au niveau dudit masque respiratoire.
